# EUROPEAN PATENT SPECIFICATION

(11) **EP 4 226 957 B1**
(45) Date of publication and mention of the grant of the patent: **25.11.2026**
(21) Application number: 22156022.0
(22) Date of filing: 10.02.2022
(51) Int. Cl.: A61M 5/142

(54) **BODY-WEARABLE MEDICAL DEVICE COMPRISING A SEALING ELEMENT**
AM KÖRPER TRAGBARE MEDIZINISCHE VORRICHTUNG MIT DICHTUNGSELEMENT
DISPOSITIF MÉDICAL POUVANT ÊTRE PORTÉ SUR LE CORPS, COMPRENANT UN ÉLÉMENT D'ÉTANCHÉITÉ

(43) Date of publication of application: 16.08.2023
(73) Proprietor: F. Hoffmann-La Roche AG, 4070 Basel (CH); Roche Diabetes Care GmbH, 68305 Mannheim (DE)
(72) Inventor: LIU, Chun-Wei, Hsinchu (TW); LI, Liang Yi, Hsinchu (TW); HUISINGA, Matthias, 68305 Mannheim (DE)
(74) Representative: Pfaffmann, Lucas

(56) References cited:
- EP-B1- 1 296 598
- DE-A1- 102005 053 765
- US-A1- 2003 163 090
- US-A1- 2010 106 091
- US-A1- 2017 359 924
- US-B2- 10 154 617
- US-B2- 7 510 544

## Description

The present invention relates to a body-wearable medical device comprising a first housing portion and a second housing portion, the first housing portion having a female portion and the second housing portion having a male portion, the male portion is at least partially inserted into the female portion thereby defining a circumferential clearance gap, wherein a circumferential sealing element is arranged in the circumferential clearance gap between the female portion and the male portion.

A body-wearable medical device is a small, relatively lightweight device that during its use is carried along by a patient. Thus, the body-wearable medical device is exposed to the same various environmental conditions and stress as the patient. Sometimes the body-wearable medical device is concealed by clothing, sometimes not. In order to prevent a malfunction caused by an ingress of dust, water, and/or humidity the circumferential sealing provided within the circumferential gap between the female portion and the male portion.

However, as the body-wearable medical device is carried by a user, the body-wearable medical device may not only be exposed to dust, water and humidity, but also to forces and stress caused by the patient's movements and activities. For example, during sport or movements of the user forces and stress exert on the body-wearable medical device that may cause a temporary deformation of the first housing portion and/or the second housing portion. Due to the temporary deformation of the first housing portion and/or the second housing portion, the width of the circumferential clearance gap between the female portion and the male portion may alter. A change of the width of the clearance gap may negatively affecting the sealing between the female portion and the male portion as provided by the sealing element.

US 2017/0359924 A1 is directed to an integral, metallic joint based on electrode position. An electronic assembly, including an encasement joined from at least two casing parts, wherein at least one gap region between two mutually adjoining casing parts is hermetically sealed by a metal layer that is electrodeposited onto the sections of the adjoining casing parts abutting the gap region and bridges the gap region.

Accordingly, it is an object of the present invention to provide a body-wearable medical device comprising an improved sealing that is less prone to failure by stress and forces exerting on the body-wearable medical device.

At least one of the above objects is solved by a body-wearable medical device as outlined above, wherein the female portion and the male portion are fixed to each other by a circumferential fixation glue that is arranged in the circumferential clearance gap between the female portion and the male portion next to the circumferential sealing element.

The circumferential fixation glue helps to keep the position of the first housing portion relative to the second housing portion. The circumferential fixation glue at least partially fills the clearance gap between the female portion and the male portion. Furthermore, the circumferential fixation glue helps to prevent that part of the female portion and part of the male portion are displaced from each other by stress or forces exerting on the body-wearable medical device. Thereby, the clearance gap is less likely deformed and the sealing element maintains its sealing function.
he sealing element is a sealing glue having a smaller elastic modulus than the fixation glue. The purpose of the fixation glue is to keep the female portion and the male portion within their relative position, while the sealing glue prevents an ingress of at least one of water, dust, splash water and humidity. When providing a sealing element formed by a sealing glue, the sealing glue itself helps to keep the female portion and the male portion within their relative position. Furthermore, the flexibility of the sealing glue helps to balance some deformations of the clearance gap that may be caused by stress and/or forces acting on the body-wearable medical device.

In an embodiment, the sealing glue is a polyurethane adhesive. In an embodiment, the polyurethane adhesive has a hardness of more than or equal to Shore A40 and lower than or equal to Shore A50. For example, the polyurethane adhesive has a hardness of Shore A42. According to an embodiment, the polyurethane adhesive has a viscosity of more than or equal to 30.000 millipascal-second, preferable more than or equal to 40.000 mPas, but lower than or equal to 90.000 mPas, preferably lower than or equal to 80.000 mPas.

In an embodiment, the fixation glue is a low odor acrylic adhesive. In an embodiment, at room temperature the full cure time is 8 to 24 hours, and having a shear strength of more than or equal to 20.000 MPa and lower than or equal to 30.000 MPa. For example, the acrylic adhesive can have a shear strength of 28.959 MPa.

In an embodiment, the sealing element comprises an O-ring that is arranged in the circumferential gap between the female portion and the male portion and that circumscribes the male portion. The O-ring is an elastic plastic ring circumscribing the male portion within the circumferential gap between the female portion and the male portion. The O-ring can form the sealing element as a standalone element, but can also form the sealing element together with the sealing glue. When being used together with the sealing glue, the O-ring is a reinforcement of the sealing element while the sealing glue helps to keep the sealing element in close sealing contact with the male portion and the female portion. The O-ring may additionally or alternatively form a barrier that is arranged next to the fixation glue and that helps to prevent the sealing glue from futher ingressing into the housing.

In an embodiment, the fixation glue discontinuously circumscribes the male portion in the circumferential clearance gap between the female portion and the male portion. The fixation glue can continuously or discontinuously circumscribe the male portion at least partially filling the clearance gap between the male portion and the female portion. In the meaning of the present invention, a discontinuously circumscribing fixation glue comprises multiple distinct fixation glue elements that are separated from each other along the circumference of the male portion within the clearance gap. Each of the distinct fixation glue elements is in contact with the male portion and the female portion thereby fixing the male portion and the female portion together. According to an embodiment, the sum of length of each of the distinct fixation glue elements is larger than 40% of the circumferential length of the male portion along the line of the distinct fixation glue elements. In an embodiment, the sum of length of each of the distinct fixation glue elements is larger than 50% of the circumferential length of the male portion along the line of the distinct fixation glue elements. The sum of length of each of the distinct fixation glue elements can be smaller than 80% or 70% of the circumferential length of the male portion along the line of the distinct fixation glue elements.

In an embodiment, the discontinuously circumscribing fixation glue is evenly distributed along the circumference of the male portion. Accordingly, the distinct fixation glue elements are evenly distributed along the circumferential length of the male portion within the clearance gap.

In an embodiment, the circumferential clearance gap is formed by a circumferential recess of the male portion and/or a circumferential recess of the female portion. Accordingly, the clearance gap can be formed by a recess in the male portion that extends along the circumference of the male portion. Alternatively, the clearance gap can be formed by a recess in the female portion that extends along the inner circumference of the female portion. Further alternatively, the clearance gap can be formed by both, a recess in the female portion extending along the inner circumference of the female portion facing the male portion and a recess in the male portion extending along the outer circumference of the male portion facing the female portion.

According to an embodiment, the male portion comprises a free-end portion facing away from the first housing portion, wherein the sealing element is located closer to the free-end portion than the fixation glue. In an order from the free-end towards the insertion direction of the male portion, the sealing element is arranged closer to the free-end portion than the fixation glue. The sealing element forms an outer boundary while the fixation glue is arranged inward relative to the sealing element. As the male portion is at least partially inserted into the female portion, the free-end portion of the male portion can projects out of the female portion or is flush with the female portion. As mentioned earlier, the sealing element can comprise a sealing glue or can be formed by a sealing glue.

In an embodiment, the first housing portion and the second housing portion form a watertight compartment. By forming a watertight compartment the sealing element forms a watertight sealing preventing an ingress of water through the clearance gap between the male portion and the female portion.

In an embodiment, in a cross-sectional view, the circumferential clearance gap forms a circular, a rectangular, a quadratic or elliptic shape circumscribing the male portion. The respective circular, rectangular, quadratic or elliptic shape circumscribing the male portion is formed by an inner wall portion of the female portion that faces the male portion. When being viewed in a cross-sectional view, the inner wall portion of the female portion forms an outer boundary of the circumferential clearance gap.

In an embodiment, the rectangular or elliptic shape that, in a cross-sectional view of the body-wearable medical device, is formed by the circumferential clearance gap comprises a first extension in one direction and a second extension in a second direction, wherein the first extension is at least 1.5 times larger than the second extension in the second direction. In an embodiment, the first extension is at least 2 times larger than the second extension in the second direction. In an embodiment, the first extension is at least 3 times larger than the second extension in the second direction. The first direction and second direction are perpendicular to each other.

According to an embodiment, the body-wearable medical device is a drug delivery device, in particular an insulin delivery device, such as for example an insulin pen or an insulin infusion pump. The body-wearable medical device can be a continuous glucose monitoring device or a body-wearable medical device that is configured for analyzing bodily fluids. The body-wearable medical device can comprise a plaster and/or an adhesive layer that is configured for temporarily adhering the body-wearable medical device to a patient's body. The body-wearable medical device can comprise a transcutaneous element such as for example a cannula, a probe or a sensor. The probe configured for retrieving bodily fluids, while the sensor is configured for analyzing bodily fluids.

Further advantages, features and technical effects of the present invention are apparent from the following description of embodiments and the accompanying figures.
- Figure 1: shows a schematic longitudinal view of a body-wearable medical device according to an embodiment of the present invention;
- Figure 2: shows a schematic cross-sectional view of the body-wearable medical device according to figure 1;
- Figure 3: shows a schematic longitudinal view of a body-wearable medical device according to a further embodiment of the present invention; and
- Figure 4: shows a schematic cross-sectional view of the body-wearable medical device according to figure 3.

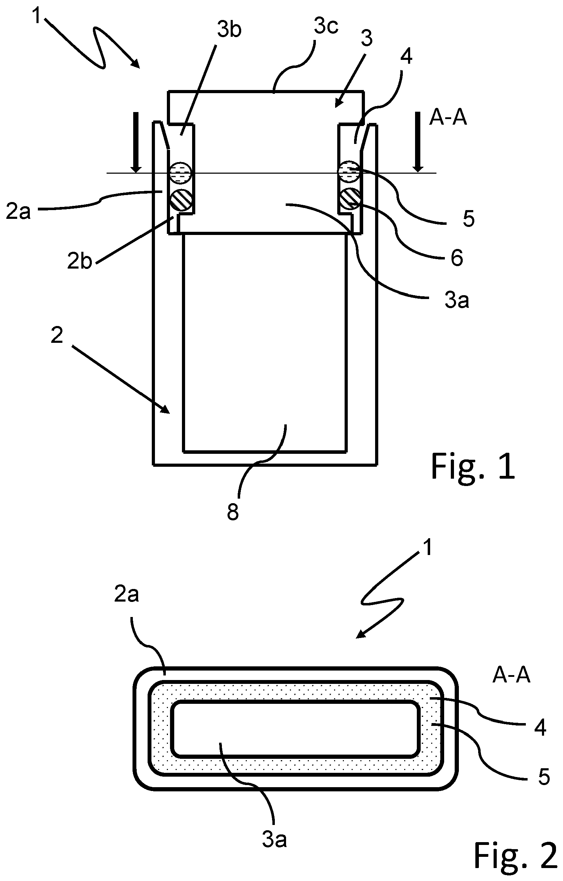

Figures 1 and 2 show a body-wearable medical device 1 according to a first embodiment of the present invention. Figure 1 shows a schematic longitudinal view of the body-wearable medical device 1, while figure 2 shows a schematic cross-sectional view of the body-wearable medical device 1 along line A-A.

The body-wearable medical device 1 comprises a first housing portion 2 and a second housing portion 3. The first housing portion 2 comprises a female portion 2a and the second housing portion 3 comprises a male portion 3a. The male portion 3a is at least partially inserted into the female portion 2a, i.e. the female portion 2a forms a receiving portion for accommodating the male portion 3a of the second housing portion 3.

A clearance gap 4 is provided between the male portion 3a and the female portion 2a, wherein the clearance gap 4 circumscribes the male portion 3a. Here in this embodiment, the clearance gap 4 is at least partially formed by a circumferential recess 3b of the male portion 3a and a circumferential recess 2b of the female portion 2a. The circumferential recess 3b of the male portion 3a and the circumferential recess 2b of the female portion 2a are facing each other.

The female portion 2a and the male portion 3a are fixed to each other by a fixation glue 6 that is evenly distributed within the clearance gap 4 along the circumference of the male portion 3a. Next to the fixation glue a sealing element 5, here a sealing glue, is provided within the clearance gap 4. The sealing element 5 is continuously distributed along the circumference of the male portion 3a and in sealing contact with the female portion 2a and the male portion 3a.

The second housing portion 3, i.e. the housing portion comprising the male portion 3a, comprises a free-end 3c portion that is facing away from the first housing portion 2, i.e. the housing portion comprising the female portion 2a. The sealing element 5 is arranged closer to the free-end 3c portion than the fixation glue 6. In a direction of insertion of the male portion 3a into the female portion 2b, the sealing element 5 forms an outer boundary while the fixation glue 6 forms an inner fixation means for holding the male portion 3a in fixed position relative to the female portion 2a.

The first housing portion 2 and the second housing portion 3 together form a watertight compartment 8.

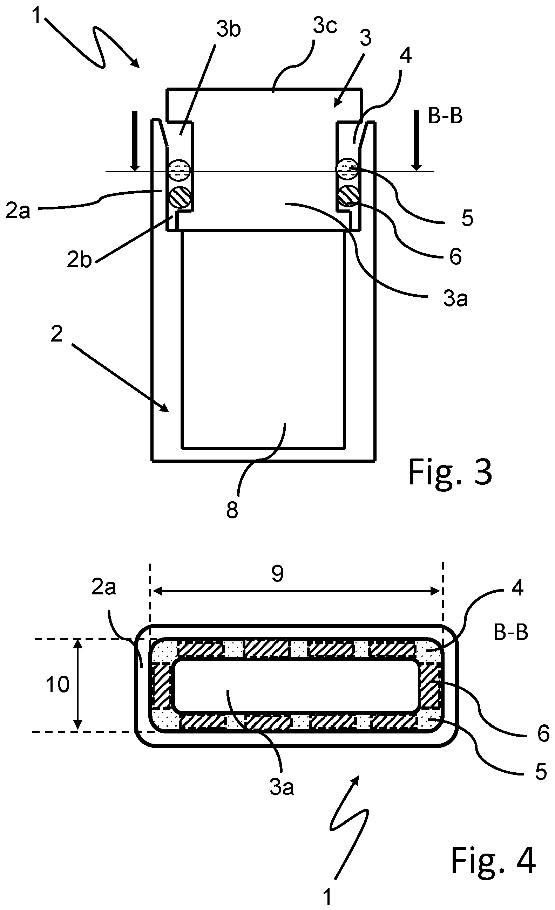

A second embodiment of a body-wearable medical device 1 is shown by figures 3 and 4. Figure 3 shows a longitudinal view of the body-wearable medical device 1, while figure 4 shows a cross-sectional view of the body-wearable medical device 1 along line B-B.

The body-wearable medical device 1 according figures 3 and 4 differs from the body-wearable medical device 1 according to the embodiment of figures 1 and 2 by having a different configuration of the fixation glue 6.

The fixation glue 6 discontinuously circumscribes the male portion 3a within the circumferential gap 4 between the female portion 2a and the male portion 3a. The fixation glue 6 is formed by multiple fixation glue elements that are evenly distributed along the circumference of the male portion 3a. The multiple fixation glue elements forming the fixation glue 6 are indicated in the cross-sectional view of figure 4 in broken line for illustrative purposed only as the fixation glue elements are arranged underneath the sealing element 5 and would not be visible in the cross-section along line B-B.

The circumferential clearance gap 4 forms a rectangular-like shape having a first extension 9 in a first direction and a second extension 10 in a second direction. The first extension is at least 1.5 times, here 2 times, larger than the second extension 10 in the second direction. The first direction and the second direction are perpendicular to each other.

### List of reference numbers

- 1: body-wearable medical device
- 2: first housing portion
- 2a: female portion
- 2b: circumferential recess of female portion
- 3: second housing portion
- 3a: male portion
- 3b: circumferential recess of male portion
- 3c: free-end portion of male portion
- 4: circumferential clearance gap
- 5: circumferential sealing element
- 6: circumferential fixation glue
- 7: O-ring
- 8: compartment
- 9: first extension in first direction
- 10: second extension in second direction

## Claims

1. A body-wearable medical device (1) comprising a first housing portion (2) and a second housing portion (3), the first housing portion (2) having a female portion (2a) and the second housing portion (3) having a male portion (3a), the male portion (3a) is at least partially inserted into the female portion (2a) thereby defining a circumferential clearance gap (4), wherein a circumferential sealing element (5) is arranged in the circumferential clearance gap (4) between the female portion (2a) and the male portion (3a),
wherein
the female portion (2a) and the male portion (3a) are fixed to each other by a circumferential fixation glue (6) that is arranged in the circumferential clearance gap (4) between the female portion (2a) and the male portion (3a) next to the circumferential sealing element (5), **characterized in that** the sealing element (5) is a sealing glue having a smaller elastic modulus than the fixation glue (6).

2. Body-wearable medical device (1) according to claim 1, **characterized in that** the sealing element (5) comprises the sealing glue having a smaller elastic modulus than the fixation glue (6), and an O-ring (7) that is arranged in the circumferential clearance gap (4) between the female portion (2a) and the male portion (3a) and that circumscribes the male portion (3a).

3. Body-wearable medical device (1) according to any one of claims 1 or 2, **characterized in that** the fixation glue (6) discontinuously circumscribes the male portion (3a) within the circumferential clearance gap (4) between the female portion (2a) and the male portion (3a).

4. Body-wearable medical device (1) according to claim 3, **characterized in that** the discontinuously circumscribing fixation glue (6) is evenly distributed along the circumference of the male portion (3a).

5. Body-wearable medical device (1) according to any one of claims 1 to 4, **characterized in that** the circumferential clearance gap (4) is formed by a circumferential recess (3b) of the male portion (3a) and/or a circumferential recess (2b) of the female portion (2a).

6. Body-wearable medical device (1) according to any one of claims 1 to 5, **characterized in that** the second housing portion (3) comprises a free-end portion (3c) facing away from the first housing portion (2), wherein the sealing element (5) is located closer to the free-end portion (3c) than the fixation glue (6).

7. Body-wearable medical device (1) according to any one of claims 1 to 6, **characterized in that** the first housing portion (2) and the second housing portion (3) form a watertight compartment (8).

8. Body-wearable medical device (1) according to any one of claims 1 to 7, **characterized in that**, in a cross-sectional view, the circumferential clearance gap (4) forms a rectangular or elliptic shape circumscribing the male portion (3a).

9. Body-wearable medical device (1) according to claim 8, **characterized in that** the rectangular or elliptic shape comprises a first extension (9) in one direction and a second extension (10) in a second direction, wherein the first extension (9) is at least 1.5 times larger than the second extension (10) in the second direction.

## Patentansprüche

1. Am Körper tragbare medizinische Vorrichtung (1), umfassend einen ersten Gehäuseteil (2) und einen zweiten Gehäuseteil (3), wobei der erste Gehäuseteil (2) einen Aufnahmeteil (2a) und der zweite Gehäuseteil (3) einen Steckteil (3a) aufweist, wobei der Steckteil (3a) zumindest teilweise in den Aufnahmeteil (2a) eingeführt ist, wodurch ein umlaufender Spalt (4) gebildet wird, wobei in dem umlaufenden Spalt (4) zwischen dem Aufnahmeteil (2a) und dem Steckteil (3a) ein umlaufendes Dichtungselement (5) angeordnet ist,
wobei
der Aufnahmeteil (2a) und der Steckteil (3a) durch einen umlaufenden Befestigungsklebstoff (6) aneinander befestigt sind, der in dem umlaufenden Spalt (4) zwischen dem Aufnahmeteil (2a) und dem Steckteil (3a) neben dem umlaufenden Dichtungselement (5) angeordnet ist, **dadurch gekennzeichnet, dass** das Dichtungselement (5) ein Dichtungsklebstoff mit einem kleineren Elastizitätsmodul als der Befestigungsklebstoff (6) ist.

2. Am Körper tragbare medizinische Vorrichtung (1) nach Anspruch 1, **dadurch gekennzeichnet, dass** das Dichtungselement (5) den Dichtungsklebstoff mit einem kleineren Elastizitätsmodul als der Befestigungsklebstoff (6) und einen O-Ring (7) umfasst, der in dem umlaufenden Spalt (4) zwischen dem Aufnahmeteil (2a) und dem Steckteil (3a) angeordnet ist und der den Steckteil (3a) umschließt.

3. Am Körper tragbare medizinische Vorrichtung (1) nach einem der Ansprüche 1 oder 2, **dadurch gekennzeichnet, dass** der Befestigungsklebstoff (6) den Steckteil (3a) innerhalb des umlaufenden Spalts (4) zwischen dem Aufnahmeteil (2a) und dem Steckteil (3a) diskontinuierlich umschließt.

4. Am Körper tragbare medizinische Vorrichtung (1) nach Anspruch 3, **dadurch gekennzeichnet, dass** der diskontinuierlich umschließende Befestigungsklebstoff (6) gleichmäßig entlang des Umfangs des Steckteils (3a) verteilt ist.

5. Am Körper tragbare medizinische Vorrichtung (1) nach einem der Ansprüche 1 bis 4, **dadurch gekennzeichnet, dass** der umlaufende Spalt (4) durch eine umlaufende Ausnehmung (3b) des Steckteils (3a) und/oder eine umlaufende Ausnehmung (2b) des Aufnahmeteils (2a) gebildet ist.

6. Am Körper tragbare medizinische Vorrichtung (1) nach einem der Ansprüche 1 bis 5, **dadurch gekennzeichnet, dass** der zweite Gehäuseteil (3) einen dem ersten Gehäuseteil (2) abgewandten freien Endteil (3c) aufweist, wobei das Dichtungselement (5) näher an dem freien Endteil (3c) angeordnet ist als der Befestigungsklebstoff (6).

7. Am Körper tragbare medizinische Vorrichtung (1) nach einem der Ansprüche 1 bis 6, **dadurch gekennzeichnet, dass** der erste Gehäuseteil (2) und der zweite Gehäuseteil (3) eine wasserdichte Kammer (8) bilden.

8. Am Körper tragbare medizinische Vorrichtung (1) nach einem der Ansprüche 1 bis 7, **dadurch gekennzeichnet, dass** der umlaufende Spalt (4) in einer Querschnittsansicht eine rechteckige oder elliptische Form bildet, die den Steckteil (3a) umschließt.

9. Am Körper tragbare medizinische Vorrichtung (1) nach Anspruch 8, **dadurch gekennzeichnet, dass** die rechteckige oder elliptische Form eine erste Ausdehnung (9) in einer Richtung und eine zweite Ausdehnung (10) in einer zweiten Richtung umfasst, wobei die erste Ausdehnung (9) mindestens 1,5-mal größer ist als die zweite Ausdehnung (10) in der zweiten Richtung.

## Revendications

1. Dispositif médical pouvant être porté sur le corps (1) comprenant une première partie de boîtier (2) et une seconde partie de boîtier (3), la première partie de boîtier (2) ayant une partie femelle (2a) et la seconde partie de boîtier (3) ayant une partie mâle (3a), la partie mâle (3a) est au moins partiellement insérée dans la partie femelle (2a) définissant ainsi un espace de dégagement circonférentiel (4), dans lequel un élément d'étanchéité circonférentiel (5) est agencé dans l'espace de dégagement circonférentiel (4) entre la partie femelle (2a) et la partie mâle (3a),
dans lequel
la partie femelle (2a) et la partie mâle (3a) sont fixées l'une à l'autre par une colle de fixation circonférentielle (6) qui est agencée dans l'espace de dégagement circonférentiel (4) entre la partie femelle (2a) et la partie mâle (3a) à côté de l'élément d'étanchéité circonférentiel (5), **caractérisé en ce que** l'élément d'étanchéité (5) est une colle d'étanchéité ayant un module élastique inférieur à celui de la colle de fixation (6).

2. Dispositif médical pouvant être porté sur le corps (1) selon la revendication 1, **caractérisé en ce que** l'élément d'étanchéité (5) comprend la colle d'étanchéité ayant un module élastique inférieur à celui de la colle de fixation (6), et un joint torique (7) qui est agencé dans l'espace de dégagement circonférentiel (4) entre la partie femelle (2a) et la partie mâle (3a) et qui circonscrit la partie mâle (3a).

3. Dispositif médical pouvant être porté sur le corps (1) selon l'une quelconque des revendications 1 ou 2, **caractérisé en ce que** la colle de fixation (6) circonscrit de manière discontinue la partie mâle (3a) au sein de l'espace de dégagement circonférentiel (4) entre la partie femelle (2a) et la partie mâle (3a).

4. Dispositif médical pouvant être porté sur le corps (1) selon la revendication 3, **caractérisé en ce que** la colle de fixation (6) qui circonscrit de manière discontinue est répartie uniformément le long de la circonférence de la partie mâle (3a).

5. Dispositif médical pouvant être porté sur le corps (1) selon l'une quelconque des revendications 1 à 4, **caractérisé en ce que** l'espace de dégagement circonférentiel (4) est formé par un évidement circonférentiel (3b) de la partie mâle (3a) et/ou un évidement circonférentiel (2b) de la partie femelle (2a).

6. Dispositif médical pouvant être porté sur le corps (1) selon l'une quelconque des revendications 1 à 5, **caractérisé en ce que** la seconde partie de boîtier (3) comprend une partie d'extrémité libre (3c) orientée à l'opposé de la première partie de boîtier (2), dans lequel l'élément d'étanchéité (5) est situé plus près de la partie d'extrémité libre (3c) que la colle de fixation (6).

7. Dispositif médical pouvant être porté sur le corps (1) selon l'une quelconque des revendications 1 à 6, **caractérisé en ce que** la première partie de boîtier (2) et la seconde partie de boîtier (3) forment un compartiment étanche à l'eau (8).

8. Dispositif médical pouvant être porté sur le corps (1) selon l'une quelconque des revendications 1 à 7, **caractérisé en ce que**, dans une vue en coupe transversale, l'espace de dégagement circonférentiel (4) forme une forme rectangulaire ou elliptique qui circonscrit la partie mâle (3a).

9. Dispositif médical pouvant être porté sur le corps (1) selon la revendication 8, **caractérisé en ce que** la forme rectangulaire ou elliptique comprend un premier allongement (9) dans une direction et un second allongement (10) dans une seconde direction, dans lequel le premier allongement (9) est au moins 1,5 fois plus grand que le second allongement (10) dans la seconde direction.
